# EUROPÄISCHE PATENTSCHRIFT

(11) **EP 1 609 204 B1**
(45) Veröffentlichungstag und Bekanntmachung des Hinweises auf die Patenterteilung: **19.08.2009**
(21) Anmeldenummer: 04711350.1
(22) Anmeldetag: 16.02.2004
(51) Int. Cl.: H01M 10/08, H01M 10/06, H01M 4/14

(54) **MIKROORGANISMEN ENTHALTENDER AKKUMULATOR**
ACCUMULATOR CONTAINING MICROORGANISMS
ACCUMULATEUR CONTENANT MICRO-ORGANISMES

(30) Priorität: 14.02.2003 DE 10306489
(43) Veröffentlichungstag der Anmeldung: 28.12.2005
(73) Patentinhaber: Georg Fritzmeier GmbH + Co. KG, 85655 Grosshelfendorf (DE)
(72) Erfinder: UPHOFF, Christian, 83229 Aschau i. Chiemgau (DE)
(74) Vertreter: Winter, Brandl, Fürniss, Hübner Röss, Kaiser, Polte Partnerschaft Patent- und Rechtsanwaltskanzlei
(86) Internationale Anmeldenummer: PCT/EP2004/001444
(87) Internationale Veröffentlichungsnummer: WO 2004/073083

(56) Entgegenhaltungen:
- US-A- 4 085 254

## Beschreibung

Die Erfindung betrifft einen Akkumulator gemäß dem Oberbegriff des Patentanspruchs 1 sowie ein Verfahren zum Herstellen eines Akkumulators.

Derartige Akkumulatoren finden beispielsweise in Kraftfahrzeugen oder Flurförderfahrzeugen Anwendung. Herkömmliche Autobatterien haben eine Blei-Kathode und eine Bleidioxidanode, die in einem Elektrolyten bestehend aus verdünnter Schwefelsäure aufgenommen sind. Beim Entladen bildet sich an den Elektroden Bleisulfat, das beim Ladevorgang der Batterie (Akkumulator) wieder in Blei bzw. Bleidioxid umgewandelt wird. Nach längerem Gebrauch werden die Sulfatkristalle beim Ladevorgang nicht mehr vollständig abgebaut, so dass an den Elektroden allmählich grobe Sulfat-Kristalle wachsen. Weitere Faktoren, die eine vorzeitige Alterung der Batterie unterstützen, sind seltene Vollladungen, eine hohe Entladetiefe und Säureschichtungen. Letztere sind in Batterien mit flüssigen Elektrolyten einer der wichtigsten Alterungsfaktoren.

Einhergehend mit einer zyklischen Belastung und / oder niedrigen Temperaturen kommt es zudem zu einer Korrosion in der Batterie. Diese führt zu einer Erhöhung es Innenwiderstands und zur Abnahme des Wirkungsgrads. Die Korrosion des positiven Gitters, hervorgerufen durch das Sulfatkristallwachstum, führt zur Abschlammung und somit zum Verlust der aktiven Masse mit der Folge von Mikrokurzschlüssen. Das Ergebnis ist eine Selbstentladung, welche wiederum zur Abnahme des Wirkungsgrad führt.

Durch diesen Alterungsprozess der Batterie wird deren Kapazität wesentlich verringert, so dass insbesondere bei niedrigen Aussentemperaturen nicht mehr genug Spannung vorhanden ist, um den Anlasser zu betätigen.

Entsprechende Alterungsprozesse laufen auch bei Sekundärelementen (Akkumulatoren) ab, bei denen andere Elektroden- und/oder Elektrolytzusammensetzungen Einsatz finden und bei denen sich nach längerem Gebrauch ein unerwünschtes Kristallwachstum an den Elektroden einstellt.

Beispielsweise offenbart die amerikanische Patentschrift US 4,085,254 eine elektrische Energiequelle mit einem Elektrodenpaar, wobei zwischen den Elektroden ein Elektrolyt eingebracht ist. Der Elektrolyt enthält koexistente aerobe und anaerobe Bakterien, die sicherstellen, dass der Elektrolyt nicht als Flüssigkeit vorliegt. Durch diese im Wesentlichen trockene elektrische Energiequelle wird die Lebenserwartung der Energiequelle verlängert, da ein Auslaufen des Elektrolyts aufgrund von Leckage nicht eintreten kann. Zudem werden die Elektroden vor dem Zusammenbau der Energiequelle in Phosphorsäure getaucht, um eine Oxidation des Elektrodenmaterials zu verzögern und dadurch ebenfalls die Lebensdauer der Energiequelle zu verlängern.

Dennoch stellt sich auch bei dieser offenbarten Energiequelle über die Zeit ein Kristallwachstum an den Elektroden ein, wodurch der Alterungsprozess aufgrund von Kristallwachstum nicht verhindert wird.

Unter www.novitec.de wird ein Batteriepulser beschrieben, bei dem mittels eines elektronischen Steuergerätes während des Ladevorganges Impulse an die Batterie mit der atomaren Resonanz-Frequenz der Sulfatkristalle (3,26 MHz) abgegeben werden. Durch diese Impulse werden die Sulfatkristalle aufgelöst und somit der Alterungsprozess der Batterie verhindert oder zumindest verlangsamt.

Nachteilig bei dieser Lösung ist, dass das Steuergerät verhältnismäßig teuer ist und dass ein hoher Aufwand betrieben werden muss, um das Steuergerät an die Batterie anzuschließen und im Motorraum zu installieren.

Dem gegenüber liegt der Erfindung die Aufgabe zugrunde, einen Akkumulator zu schaffen, bei dem der vorgeschriebene Alterungsprozess mit minimalem vorrichtungstechnischen Aufwand verlangsamt oder verhindert ist.

Diese Aufgabe wird durch einen Akkumulator mit den Merkmalen des Patentanspruchs 1 sowie durch ein Verfahren zum Herstellen eines derartigen Akkumulators gelöst.

Erfindungsgemäß wird dem Elektrolyten des Akkumulators eine mikrobiotische Mischung hinzugefügt, die in der Lage sind, die sich an den Elektroden ausbildenden unerwünschten Kristallstrukturen aufzulösen oder deren Ausbildung zu verhindern. Es zeigte sich überraschenderweise, dass derartige Mikroorganismen im Elektrolyten, beispielsweise in Schwefelsäure, überleben können. Ein besonderer Vorteil der Erfindung liegt darin, dass keinerlei zusätzliche Installationen im Motorraum erforderlich sind, da die Mikroorganismen bei der Produktion der Batterie bereits hinzugefügt werden. Prinzipiell ist es auch vorstellbar, diese Mikroorganismen nachträglich einer Batterie zuzuführen, bei der der Alterungsprozeß bereits eingesetzt hat.

Es zeigte sich, dass Mikroorganismen mit einem Anteil an Leuchtbakterien und einem Anteil photosynthetisch arbeitender Bakterien besonders vorteilhaft einsetzbar sind. Die Leuchtbakterien liefern bei einer derartigen Mischung praktisch die Energie, die die photosynthetisch arbeitenden Bakterien benötigen, um die Kristallstrukturen abzubauen.

Die Mikroorganismen sind so gewählt, dass sie im Elektrolyten von einer schleimigen extrazellulären Schutzschicht umgeben sind, die verhindert, dass die Mikroorganismen in der sauren Umgebung beschädigt werden.

Vor dem Einbringen in den Elektrolyten können in die Mikroorganismen, beispielsweise durch Gefriertrocknen, immobilisiert und stabilisiert werden.

Im erfindungsgemäßen Akkumulator können Mikroorganismen eingesetzt werden, wie sie beispielsweise in der DE 100 62 812 A1 offenbart sind.

Im folgenden wird die Wirkungsweise der Erfindung anhand einer Autobatterie erläutert.

Eine derartige Autobatterie (Bleiakkumulator) hat eine Anode aus Bleidioxid (PbO₂), eine Kathode aus reinem Blei (Pb) sowie verdünnte Schwefelsäure als Elektrolyt. Wie bereits vorstehend beschrieben, wird durch den Alterungsprozeß einer derartigen Batterie die sich im Neuzustand ausbildende schwammartige Struktur an den Elektrodenoberflächen durch Kristallisation in ein poröses, grobkristalliges Bleisulfat umgewandelt und somit die Kapazität der Batterie verringert. Bei dem erfindungsgemäßen Akkumulator wird in den Elektrolyten, beispielweise in die verdünnte Schwefelsäure eine mikrobiotische Mischung eingebracht. Diese mikrobiotische Mischung enthält einen Anteil an lichtemittierenden Mikroorganismen und einen Anteil von photosynthetisch arbeitenden Mikroorganismen. Das Licht, das die Photosynthese antreibt stammt dabei von den lichtemittierenden Mikroorganismen (Leuchtbakterien). Diese besitzen eine Leuchtkraft, d. h. sie sind in der Lage, Lichtquanten auszusenden.

Das Wechselspiel zwischen den photosynthetisch arbeitenden Mikroorganismen und den Leuchtbakterien führt dazu, dass die photosynthetisch arbeitenden Mikroorganismen durch das emittierte Licht zur Photosynthese angeregt werden. Die Mikroorganismen betreiben die Photosynthese mit Schwefelwasserstoff und Wasser als Edukt und setzen Schwefel bzw. Sauerstoff frei.

Ferner können sie Stickstoff sowie Phosphat binden und organische sowie anorganische Materie, insbesondere die sich ausbildenden Sulfatkristalle auflösen. Bevorzugt werden in der erfindungsgemäßen mikrobiologischen Zusammensetzung photosynthetisch arbeitende Mikroorganismen verwendet, die fakultativ phototroph sind. Phototroph fakultativ bedeutet, dass die Mikroorganismen sowohl unter anaeroben Bedingungen im Licht als auch unter aeroben Bedingungen im Dunklen wachsen können.

Zu den Photosynthesebakterien gehören gramnegative aerobe stabförmige und kreisförmige Bakterien sowie grampositive kreisförmige Bakterien. Diese können Endosporen aufweisen oder als andere Sporen vorhanden sein. Dazu zählen beispielsweise auch grampositive Aktinomyceten und verwandte Bakterien.

In diesem Zusammenhang können auch stickstoffbindende Organismen genannt werden. Dazu gehören beispielweise Algen, wie Anabena Nostoc in Symbiose mit Azola. Des Weiteren können Aktinomyceten, z. B. Frankia in Symbiose mit Erlen und Bakterien, wie Rhizobium in Symbiose mit mit Leguminosen, erwähnt werden.

Außerdem können auch aerobe Algen, Azotobacter, methanoxidierende Bakterien und Schwefelbakterien verwendet werden. Dazu zählen auch grüne Schwefelbakterien und braungrüne Photosynthesebakterien. Hier können auch nicht violette Schwefelbakterien und violette Schwefelbakterien genannt werden. Des Weiteren enthält die Mischung Bakterien des Typs Geobacter Sulfurreducencs.

Es ist bevorzugt, dass in der erfindungsgemäßen mikrobiologischen Zusammensetzung als fakultativ phototrophe Mikororganismen, Prochlorophyten, Cyanobakterien, grüne Schwefelbakterien, Purpurbakterien, Chloroflex-us-ähnliche Formen und Heliobakterum und

Heliobacillus-ähnliche Formen enthalten sind. Die vorgenannten fakultativ phototrophen Mikroorganismen können auch als Mischungen aus zwei oder mehr davon vorliegen. In einer Ausführungsform liegen alle sechs genannten Mikroorganismen als Mischung vor.

In der erfindungsgemäßen Mischung können als Leuchtbakterien Photobacterium phosphoreum, Vibrio fischen, Vibrio harveyi, Pseudomonas lucifera oder Beneckea enthalten. Es ist auch möglich, eine Mischung aus mindestens zwei daraus zu wählen.

Zur Optimierung der erfindungsgemäßen mikrobiologischen Zusammensetzung können weitere Bestandteile darin enthalten sein. Vorzugsweise sind solche Nebenbestandteile Pflanzenextrakte, Enzyme, Spurenelemente, Polysaccharide, Alginderivate, andere Mikroorganismen wie oben. Die Nebenbestandteile können einzeln oder in Kombination in der erfindungsgemäßen mikrobiologischen Zusammensetzung vorliegen.

Problematisch bei der erfindungsgemäßen Lösung ist, dass die Mikroorganismen so zur Verfügung gestellt werden müssen, dass sie in der sauren Umgebung überleben können. Erfindungsgemäß sind die Mikroorganismen derart ausgewählt, dass sich beim oder vor dem Einbringen in den Elektrolyten eine schleimartige extracelluläre polymere Substanz (EPS) bildet, in der die Mikroorganismen eingebettet sind und die diese vor Beschädigung schützt. Durch diese Schleimbildung wird eine Art Schutzschild gegen toxische Substanzen (Elektrolyt, Blei) gebildet, das ein Vordringen dieser Substanzen in das Zellinnere verhindert. Diese von der EPS umgebenen Mikroorganismen setzen sich an den Elektroden als Biofilm ab. Bei dem sich einstellenden Stoffwechselprozess werden die photosysthetisch arbeitenden Mikrooganismen durch die in der mikrobiotischen Mischung enthaltenen Leuchtbakterien durch Aussendung von Lichtquanten zur Photosynthese angeregt und dabei das sich einstellende grobkristallige, poröse Bleisulfat abgebaut, so dass sich an den Elektroden die ursprüngliche, feinporige Schwammstruktur mit Bleioxidschaum ausbildet. Dieser Prozess lässt sich auch durch nachträgliches Einfügen von Mikroorganismen in gealterte Autobatterien durchführen.

Einige der verwendeten Bakterien, insbesondere der Einzeller Geobacter Sulfurreducens wirken stabilisierend auf den Alterungsprozess. Es zeigte sich, dass Gene dieser Bakterien den Transport von Elektronen auf Metallionen ermöglichen, so dass die Metallionen durch diese Reduktion unlöslich werden. Dieser Elektronentransport erzeugt zudem Strom, der den Wirkungsgrad der Batterie erhöhen kann. D. h., diese Bakterien wirken als eine Art Biosensor und lebende Batterien.

Bei typischer Belastung produziert die Mischung von Mikroorganismen in der lastfreien Phase Enzyme, die Methanol aufspalten können. Die dabei freigesetzten Elektronen nutzt die Batterie, hohe Entladungstiefen werden weitgehend verhindert.

In der Lastphase setzt die Mischung Wasserstoff frei. Dieser wird in der lastfreien Phase unter Zufuhr (Kationen) von Energie ionisiert, wobei Protonen freigesetzt werden. Der Wasserstoff dient hierbei als temporärer Energiespeicher.

Es ist zu erwarten, dass durch die Zugabe der Mischung von Mikroorganismen zum einen die Lebensdauer der Batterie um zumindest 30 bis 40 % steigen wird, des Weiteren dürfte sich das spontane Leistungsangebot der Batterie deutlich erhöhen.

Falls die Mikroorganismen bereits im Neuzustand dem Elektrolyten hinzugegeben werden, wird das übermäßige Kristallwachstum im Bereich der Elektroden verringert oder entstehende Grobkristalle aufgelöst, so dass die mit der mikrobiotischen Mischung versehenen Akkumulatoren eine wesentlich längere Lebensdauer als herkömmliche Akkus aufweisen. Die Mehrkosten für die erfindungsgemäße Aufbereitung der Akkumulatoren sind wesentlich geringer als beim eingangs beschriebenen Stand der Technik. Die mikrobiotische Mischung lässt sich auch bei anderen Akkumulatorentypen und auch bei gelartigen Elektrolyten einsetzen.

Bei besonderen Anwendungsfällen kann es vorteilhaft sein, wenn die mikrobiotische Mischung nicht in einer Nährlösung sondern immobilisiert zugegeben wird. Eine derartige Immobilisierung der Mikroorganismen kann beispielsweise durch einen Trocknungsschritt erfolgen, bei dem die die Zellen der Mikroorganismen umgebenden extracellulären polymeren Substanzen (EPS) dehydratisiert und somit eingedickt wird und eine Schutzschicht bildet, die die Mikroorganismen während der Tiefkühl-Trocknung und auch beim Einbringen in den Elektrolyten schützt.

Offenbart ist ein Akkumulator und ein Verfahren zu dessen Herstellung, wobei einem die Elektroden des Akkumulators umgebenden Elektrolyten eine mikrobiotische Mischung zugegeben wird, über die sich ausbildende unerwünschte Kristallstrukturen verhindert oder aufgelöst werden können.

## Patentansprüche

1. Akkumulator mit einer Kathode und einer Anode, die in einem Elektrolyten angeordnet sind, **dadurch gekennzeichnet, dass** der Elektrolyt Mikroorganismen enthält, die dazu geeignet sind, die sich an der Anode oder Kathode ausbildende Kristallstrukturen aufzulösen.

2. Akkumulator nach Patentanspruch 1, wobei die Mikroorganismen einen Anteil an Leuchtbakterien und einen Anteil photosynthetisch arbeitender Bakterien enthalten.

3. Akkumulator nach Patentanspruch 1 oder 2, wobei die Kathode Blei, die Anode Bleidioxid und der Elektrolyt eine Säure, insbesondere verdünnte Schwefelsäure enthält.

4. Akkumulator nach Patentanspruch 2, wobei die Mikroorganismen mit einer schleimartigen, extracellulären Schutzschicht versehen sind.

5. Akkumulator nach einem der vorherigen Patentansprüche, wobei die Mikroorganismen das Bakterium Geobacter Sulfurreducencs enthalten.

6. Akkumulator nach einem der vorhergehenden Patentansprüche, wobei die Mikroorganismen vor dem Einbringen, vorzugsweise durch einen Trocknungsschritt, immobilisiert sind.

7. Akkumulator nach einem der vorhergehenden Patentansprüche, wobei dieser für ein Kraftfahrzeug oder ein Nutzfahrzeug, insbesondere ein Flurförderfahrzeug vorgesehen sind.

8. Verfahren zum Herstellen eines Akkumulators, der eine Anode, eine Kathode sowie einen Elektrolyten enthält, **gekennzeichnet durch** den Schritt:
Einbringen von Mikroorganismen in den Elektrolyten, wobei die Mirkoorganismen dazu ausgelegt sind, die sich an den Elektroden ausbildenden Kristallstrukturen aufzulösen.

## Claims

1. Accumulator with a cathode and an anode which are disposed in an electrolyte, **characterised in that** the electrolyte contains micro-organisms which are suitable for dissolving crystalline structures which form on the anode or cathode.

2. Accumulator as claimed in claim 1, in which the micro-organisms contain a proportion of luminescent bacteria and a proportion of photosynthetically acting bacteria.

3. Accumulator as claimed in claim 1 or 2, **characterised in that** the cathode contains lead, the anode contains lead dioxide and the electrolyte contains an acid, in particular diluted sulphuric acid.

4. Accumulator as claimed in claim 2, in which the micro-organisms are provided with a slime-like, extra-cellular protective coating.

5. Accumulator as claimed in one of the preceding claims, in which the micro-organisms contain the bacterium Geobacter sulfurreducens.

6. Accumulator as claimed in one of the preceding claims, in which the micro-organisms are immobilised before they are applied, preferably by means of a drying step.

7. Accumulator as claimed in one of the preceding claims, the latter being intended for use in a motor vehicle or a utility vehicle, in particular a field tractor.

8. Method of producing an accumulator, which contains an anode, a cathode and an electrolyte, **characterised by** the step of introducing micro-organisms into the electrolyte, the purpose of the micro-organisms being to dissolve crystalline structures forming on the electrodes.

## Revendications

1. Accumulateur avec une cathode et une anode, qui sont disposées dans un électrolyte, **caractérisé en ce que** l'électrolyte contient des micro-organismes appropriés à dissoudre les structures cristallines se formant sur l'anode ou la cathode.

2. Accumulateur selon la revendication 1, dans lequel les micro-organismes contiennent une proportion de bactéries luminescentes et une proportion de bactéries photosynthétiques.

3. Accumulateur selon la revendication 1 ou 2, dans lequel la cathode contient du plomb, l'anode contient du dioxyde de plomb et l'électrolyte contient un acide, en particulier un acide sulfurique dilué.

4. Accumulateur selon la revendication 2, dans lequel les micro-organismes sont dotés d'une couche protectrice extracellulaire de type muqueux.

5. Accumulateur selon l'une des revendications précédentes, dans lequel les micro-organismes contiennent la bactérie *Geobacter sulfurreducens.*

6. Accumulateur selon l'une des revendications précédentes, dans lequel les micro-organismes sont immobilisés avant l'introduction, de préférence par le biais d'une étape de séchage.

7. Accumulateur selon l'une des revendications précédentes, dans lequel celui-ci est prévu pour un véhicule ou un utilitaire, en particulier un chariot élévateur.

8. Procédé de fabrication d'un accumulateur, qui comprend une anode, une cathode et un électrolyte, **caractérisé par** l'étape consistant à introduire des micro-organismes dans l'électrolyte, dans lequel les micro-organismes sont conçus dans le but de dissoudre les structures cristallines se formant sur les électrodes.
